# EUROPÄISCHE PATENTSCHRIFT

(11) **EP 3 210 581 B1**
(45) Veröffentlichungstag und Bekanntmachung des Hinweises auf die Patenterteilung: **05.08.2020**
(21) Anmeldenummer: 17156392.7
(22) Anmeldetag: 16.02.2017
(51) Int. Cl.: A61F 13/06, A61F 13/08

(54) **FUSSBANDAGE ZUR KOMPRESSIONSTHERAPIE VON LYMPHÖDEMEN**
FOOT BANDAGE FOR COMPRESSION THERAPY OF LYMPHEDEMA
BANDAGE POUR PIED DESTINÉ À LA THÉRAPIE PAR COMPRESSION DES LYMPHOEDÈMES

(30) Priorität: 23.02.2016 DE 202016100937 U
(43) Veröffentlichungstag der Anmeldung: 30.08.2017
(73) Patentinhaber: Julius Zorn GmbH, 86551 Aichach (DE)
(72) Erfinder: Schettler, Uwe, 86551 Aichach (DE)
(74) Vertreter: Charrier Rapp & Liebau

(56) Entgegenhaltungen:
- EP-A1- 1 179 325
- DE-A1- 10 055 932
- DE-A1-102009 006 628
- US-A- 4 729 370
- US-A- 5 472 414

## Beschreibung

Die Erfindung betrifft eine Fußbandage zur Kompressionstherapie von Lymphödemen nach dem Oberbegriff des Anspruchs 1.

Aus der WO 2005/092401 A1 (Figur 6) ist eine Fußbandage zur Kompressionstherapie von Lymphödemen bekannt. Diese umfasst ein inneres schlauch- bzw. sockenförmiges Teil aus einem elastischen Material (Baumwoll-Lycra-Gemisch) sowie drei äußere Bänder aus einem elastischen Material. Das schlauch- bzw. sockenförmige Teil weist dabei einen Sohlenabschnitt und sich einstückig seitlich daran anschließende Seitenabschnitte auf, welche bei angelegter Bandage schlauchförmig den Mittelfuß umschließen. Zur Ausübung einer Kompression auf den Fuß werden zwei der drei elastischen Bänder um den Fußrücken gewickelt und dort mittels Befestigungsmittel unter Zug festgelegt. Das dritte Band wird um den sich proximal an den Fuß anschließenden distalen Unterschenkelbereich geschlungen und dort ebenfalls unter Zug mittels Befestigungsmittel befestigt. Aufgrund der Zugspannung der Bänder wird auf den Fuß, insbesondere im Mittelfußbereich sowie auf den distalen Unterschenkelabschnitt eine Kompression ausgeübt. Durch eine solche Kompression können Lymphödeme (Flüssigkeitsansammlungen im Zwischenzellraum, die durch mechanische Insuffizienz des Lymphgefäßsystem des menschlichen oder tierischen Körpers hervorgerufen werden) behandelt werden, indem durch den von der Fußbandage ausgeübten Kompressionsdruck der Abtransport der Lymphflüssigkeit unterstützt und dadurch der Lymphstau abgebaut wird.

Die Veröffentlichungen US 5,472,414 und US 4,729,370 zeigen Fußbandagen mit einem Grundkörper, an dem ein oberer und ein unterer Laschenabschnitt mit jeweils um den Fuß bzw. oberhalb des Sprunggelenks um das Bein wickelbare Laschen sowie mit zusätzlichen Kompressionsbändern, die am Grundkörper angenäht sind und zur Erzeugung eines Zugs auf das Sprunggelenk im Bereich oberhalb des Sprunggelenks um das Bein gewickelt und mittels Klettverschlüsse, die am freien Ende des zusätzlichen Kompressionsbands angeordnet sind, auf dem Grundkörper befestigt werden können.

Die aus dem Stand der Technik bekannte Fußbandage zur Kompressionstherapie von Lymphödemen weist den Nachteil auf, dass die Anordnung der Bänder an dem schlauch- bzw. sockenförmigen Teil fest vorgegeben ist. Daher ist eine Anpassung der Fußbandage an die Anatomie des Fußes eines Patienten nicht möglich.

Der Erfindung liegt daher die Aufgabe zugrunde, eine gattungsgemäße Fußbandage so weiterzubilden, dass eine individuelle Anpassung an die Anatomie des Fußes eines Patienten ermöglicht wird.

Diese Aufgabe wird mit einer Fußbandage mit den Merkmalen des Anspruchs 1 gelöst. Bevorzugte Ausführungsformen der Fußbandage sind den abhängigen Ansprüchen zu entnehmen.

Die erfindungsgemäße Fußbandage umfasst - wie die oben beschriebene Fußbandage des Stands der Technik - einen Sohlenabschnitt sowie zwei sich seitlich an den Sohlenabschnitt anschließende Seitenabschnitte sowie einen distalen Laschenabschnitt aus einem elastischen Material mit wenigstens einer Zuglasche, welche bei angelegter Bandage um den Fußrücken gewickelt und an einem der Seitenabschnitte befestigt ist, um eine Kompression auf den Fuß auszuüben. Der distale Laschenabschnitt umfasst dabei wenigstens eine Zuglasche, die auch - wie bei der bekannten Fußbandage - als elastisches Band ausgebildet sein kann, wobei an einem freien Ende der Zuglasche bzw. des Bands Befestigungsmittel angeordnet sind, mit denen die Zuglasche bzw. das Band unter Zug insbesondere an einem Seitenabschnitt befestigt werden kann. Weiterhin umfasst die erfindungsgemäße Fußbandage ein proximales Kompressionsband aus einem elastischen Material, welches über erste Befestigungsmittel verfügt und abnehmbar an den Seitenabschnitten der Fußbandage befestigbar ist. Bei angelegter Fußbandage wird das proximale Kompressionsband mittels der ersten Befestigungsmittel in einem Bereich oberhalb der Ferse lösbar an den Seitenabschnitten befestigt. Durch die lösbare Befestigung des proximalen Kompressionsbands an den Seitenabschnitten der Fußbandage in einem Bereich oberhalb der Ferse ist es möglich, das proximale Kompressionsband in Längsrichtung des Unterschenkels (in vertikaler Richtung) an unterschiedlichen Stellen eines distalen Unterschenkelabschnitts zu befestigen und dadurch die Befestigungsposition des proximalen Kompressionsbands an die Anatomie des Patienten anzupassen.

Zur Befestigung des proximalen Kompressionsbands unter Zug an einem distalen Unterschenkelabschnitt verfügt das proximale Kompressionsband über zweite Befestigungsmittel. Diese sind zweckmäßig an wenigstens einem Endabschnitt des proximalen Kompressionsbands angeordnet. Zur Ausübung eines Kompressionsdrucks wird das proximale Kompressionsband bei angelegter Fußbandage um den distalen Unterschenkelabschnitt gewickelt und dort unter Dehnung mittels der zweiten Befestigungsmittel festgelegt. Dabei wird zweckmäßig ein Endabschnitt des proximalen Kompressionsbands über den anderen Endabschnitt des Kompressionsbands gelegt und mittels der zweiten Befestigungsmittel auf dessen Außenseite befestigt.

Bei den Befestigungsmitteln der Zuglaschen des distalen Laschenabschnitts sowie den ersten und zweiten Befestigungsmitteln des proximalen Kompressionsbands handelt es sich zweckmäßig um Klettverschlüsse. Bevorzugt sind die Hakenteile der Klettverschlüsse dabei abnehmbar an Endabschnitten der jeweiligen Zuglaschen bzw. Bänder befestigt. Dies ermöglicht es, die Fußbandage sowohl für einen linken als auch für einen rechten Fuß zu verwenden.

In einer bevorzugten Ausführungsform verfügt jeder Seitenabschnitt der erfindungsgemäßen Fußbandage über einen distalen Laschenabschnitt, wobei jeder Laschenabschnitt wenigstens eine Zuglasche oder ein Band aufweist. Dies ermöglicht eine Verwendung der Fußbandage sowohl für einen rechten als auch für einen linken Fuß. Bevorzugt weist jeder Laschenabschnitt eine erste und mindestens eine zweite Zuglasche auf. Die Zuglaschen jedes Laschenabschnitts sind dabei zweckmäßig aus demselben elastischen Material wie das proximale Kompressionsband. Auch die Seitenabschnitte der Sohlenabschnitt sind bevorzugt aus demselben Material, welches bevorzugt ein elastisches Textilmaterial mit einer maximalen Dehnung im Bereich von 15 bis 50 % der ungedehnten Länge ist. Bei dem Textilmaterial kann es sich bspw. um ein Textilgewebe aus Polyamid und Elasthan handeln, insbesondere mit 87% Polyamid und 13% Elasthan, bezogen auf das Gewicht des Textilgewebes.

Wenn ein distaler Laschenabschnitt über mehrere Zuglaschen verfügt, sind benachbarte Zuglaschen eines Laschenabschnitts zweckmäßig durch einen Einschnitt im jeweiligen Laschenabschnitt voneinander getrennt. Dadurch ist es möglich, die Zuglaschen eines Laschenabschnitts beim Anlegen der Fußbandage winklig zueinander am gegenüberliegenden Laschenabschnitt zu befestigen. Auch dadurch kann eine Anpassung der Fußbandage an die Anatomie des Patienten erfolgen.

Weiterhin weist zweckmäßig zumindest jede Zuglasche eines der beiden distalen Laschenabschnitte ein Befestigungsmittel auf, mit dem die Zuglasche lösbar am gegenüberliegenden Seitenabschnitt bzw. dessen Laschenabschnitt befestigt werden kann. Das Befestigungsmittel kann bspw. als Hakenteil eines Klettverschlusses ausgebildet und abnehmbar an den Zuglaschen eines der beiden Laschenabschnitte angeordnet sein. Dies ermöglicht es, die Hakenteile des Klettverschlusses von den Zuglaschen des einen Laschenabschnitts zu entfernen und an den Zuglaschen des anderen Laschenabschnitts zu befestigen. Dadurch kann die Fußbandage sowohl für einen rechten als auch für einen linken Fuß verwendet werden.

In einem bevorzugten Ausführungsbeispiel sind die beiden Seitenabschnitte und der Sohlenabschnitt der Fußbandage durch separate Teile ausgebildet, welche miteinander befestigt, insbesondere vernäht sind. Dabei sind die Seitenabschnitte im Bereich der Ferse längs einer Vertikalnaht miteinander verbunden, wobei die Vertikalnaht bei angelegter Fußbandage im Bereich der Achillessehne zu liegen kommt. Jeder Seitenabschnitt ist ferner längs einer Horizontalnaht mit dem Sohlenabschnitt verbunden, insbesondere vernäht. Jeder Seitenabschnitt verfügt in einer bevorzugten Ausführungsform der erfindungsgemäßen Fußbandage über eine proximale und eine distale Kante, welche zweckmäßig jeweils zumindest im Wesentlichen gerade sind. Zwischen der proximalen Kante und der distalen Kante ist bei jedem Seitenabschnitt ein Laschenabschnitt angeordnet, der zweckmäßig über zwei oder mehr Zuglaschen oder Bänder verfügt. Die Verlängerungen der proximalen und der distalen Kante eines Seitenabschnitts treffen dabei bevorzugt unter einem Winkel aufeinander, der zwischen 40° und 50° und insbesondere bei 45° liegt.

Zur Erhöhung des Tragekomforts ist im Sohlenabschnitt zweckmäßig eine Polsterung eingearbeitet. Die Polsterung kann dabei insbesondere auf der Außenseite des Sohlenabschnitts aufgenäht sein.

Diese und weitere Merkmale und Vorteile der erfindungsgemäßen Fußbandage ergeben sich aus dem nachfolgend unter Bezugnahme auf die begleitenden Zeichnungen näher beschriebenen Ausführungsbeispiel, wobei die Zeichnungen zeigen:
- **Figur 1:**: Darstellung einer an einem Fuß angelegten Fußbandage zur Kompressionstherapie von Lymphödemen gemäß der Erfindung in einer Seitenansicht, wobei die Fußbandage ein abnehmbares proximales Kompressionsband umfasst, welches in der Darstellung von Figur 1a in geöffnetem Zustand und in der Darstellung von Figur 1b in geschlossenem Zustand gezeigt ist;
- **Figur 2:**: Schematische Darstellung der Schritte zum Anlegen der Fußbandage von Figur 1 an einen Fuß eines Patienten.

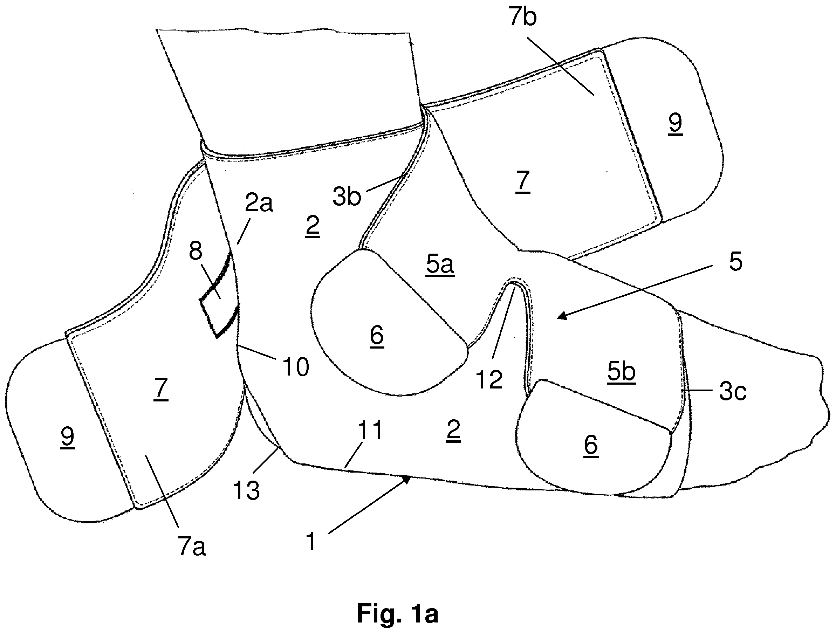

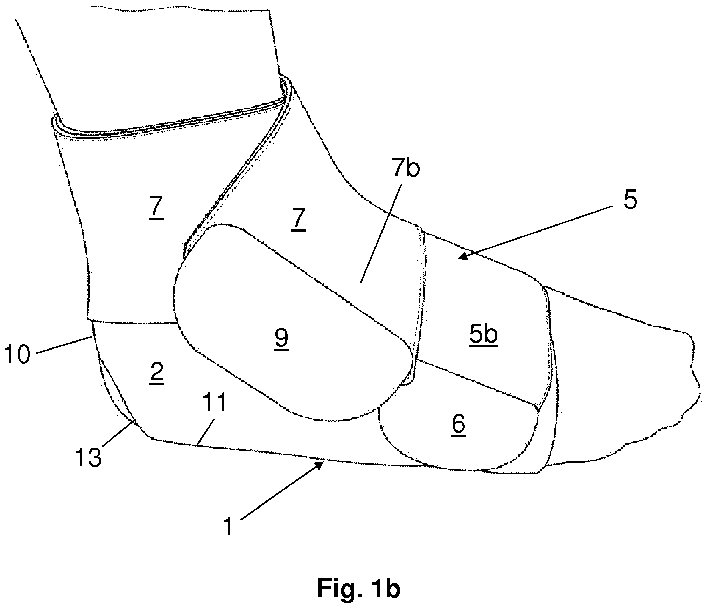

Die in Figur 1 gezeigte Fußbandage umfasst einen Sohlenabschnitt 1 und sich seitlich an den Sohlenabschnitt 1 anschließende Seitenabschnitte 2, 3. Jeder Seitenabschnitt 2, 3 ist dabei zweckmäßig längs einer Horizontalnaht 11 mit dem Sohlenabschnitt 1 verbunden, insbesondere vernäht. Es ist jedoch auch möglich, den Sohlenabschnitt 1 und die sich daran anschließenden Seitenabschnitte einstückig und damit nahtlos auszubilden.

Die beiden Seitenabschnitte 2, 3 sind in einem Bereich 2a, 3a oberhalb der Ferse miteinander durch eine Vertikalnaht 10 verbunden. Bei angelegter Fußbandage verläuft die Vertikalnaht 10 in vertikaler Richtung oberhalb der Ferse im Bereich der Achillessehne des Patienten. Im Fersenbereich weist die Fußbandage eine Ausnehmung 13 auf. Bevorzugt ist im Sohlenabschnitt 1 eine Polsterung eingearbeitet, um den Tragekomfort zu erhöhen. Die Polsterung kann beispielsweise auf einer Außenseite des Sohlenabschnitts 1 aufgenäht sein.

Jeder Seitenabschnitt 2, 3 verfügt über eine proximale Kante 2b, 3b und eine distale Kante 2c, 3c. In jedem Seitenabschnitt 2, 3 ist zwischen der proximalen Kante 2b, 3b und der distalen Kante 2c, 3c ein Laschenabschnitt 4, 5 angeordnet. Die Verlängerungen der proximalen Kante 2b, 3b und der distalen Kante 2c, 3c treffen sich dabei unter einem Winkel zwischen 40° und 50° und bevorzugt unter einem 45°-Winkel.

Der Sohlenabschnitt 1, die beiden Seitenabschnitte 2, 3 und jeder distale Laschenabschnitt 4, 5 sind dabei zweckmäßig aus einem elastischen Material gefertigt, beispielsweise einem elastischen Kurzzug-Textilmaterial. Zweckmäßig weist das elastische Material einen maximalen Dehnungsbereich zwischen 15 % und 50 % des ungedehnten Zustands auf. Bei dem elastischen Material kann es sich beispielsweise um ein Textilgewebe aus Polyamid und Elasthan, bspw. mit 87 % Polyamid und 13 % Elasthan, bezogen auf das Gewicht des Textilgewebes, handeln. Die maximale Dehnung des elastischen Materials liegt bevorzugt im Bereich von 15 % bis 50 % der ungedehnten Länge und besonders bevorzugt entweder zwischen 15 % und 20 % oder zwischen 25 % und 35 %.

Jeder distale Laschenabschnitt 4, 5 umfasst in dem zeichnerisch dargestellten Ausführungsbeispiel zwei Zuglaschen 4a, 4b bzw. 5a, 5b. Die Zuglaschen 4a, 4b bzw. 5a, 5b eines Laschenabschnitts 4, 5 sind jeweils durch einen Einschnitt 12 im jeweiligen Laschenabschnitt 4, 5 voneinander getrennt.
Zur Befestigung der Fußbandage an einem Fuß eines Patienten sind zumindest an einem der beiden distalen Laschenabschnitte 4, 5 Befestigungsmittel 6 angeordnet. Bei diesen Befestigungsmitteln 6 handelt es sich zweckmäßig um Klettverschlüsse. In dem zeichnerisch dargestellten Ausführungsbeispiel sind die Befestigungsmittel 6 jeweils abnehmbar an einem freien Ende der beiden Zuglaschen 5a, 5b des einen Laschenabschnitts 5 angeordnet.

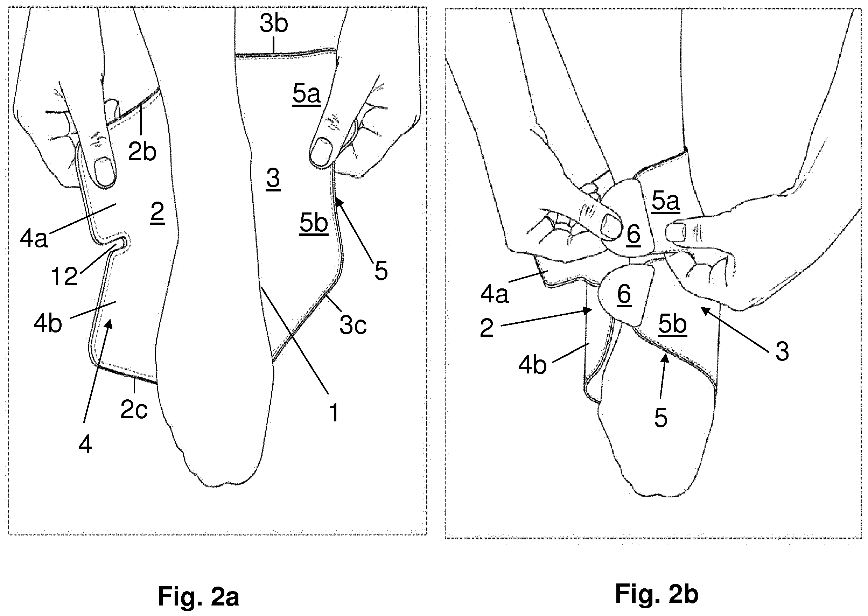

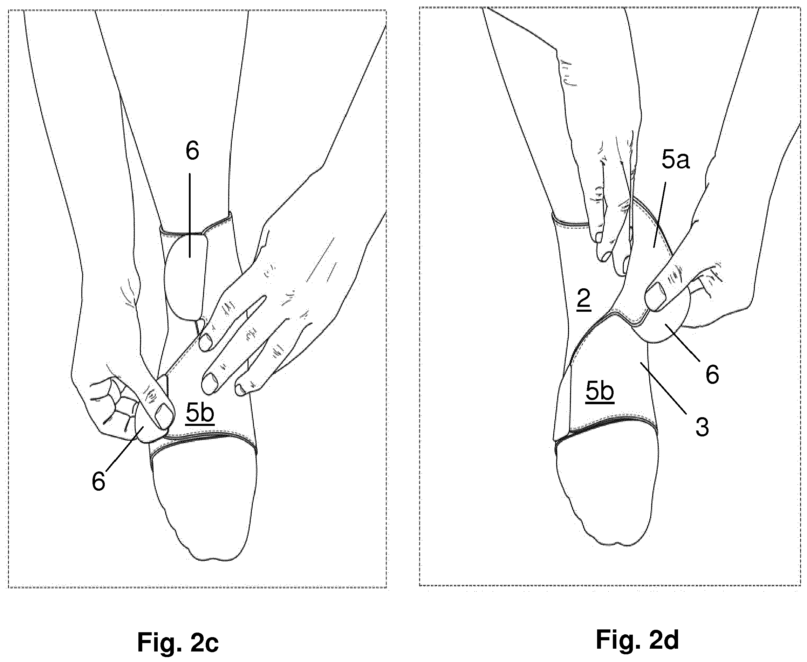

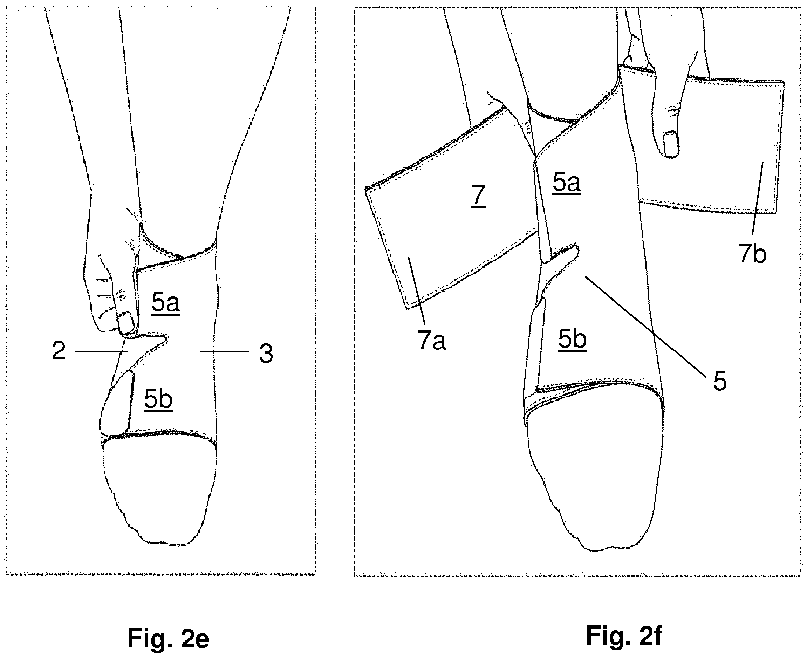

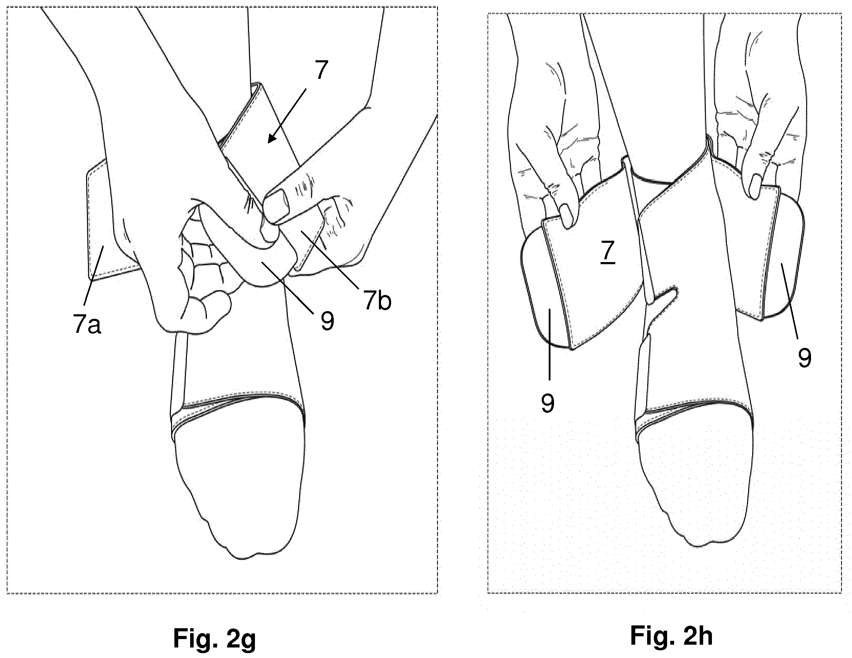

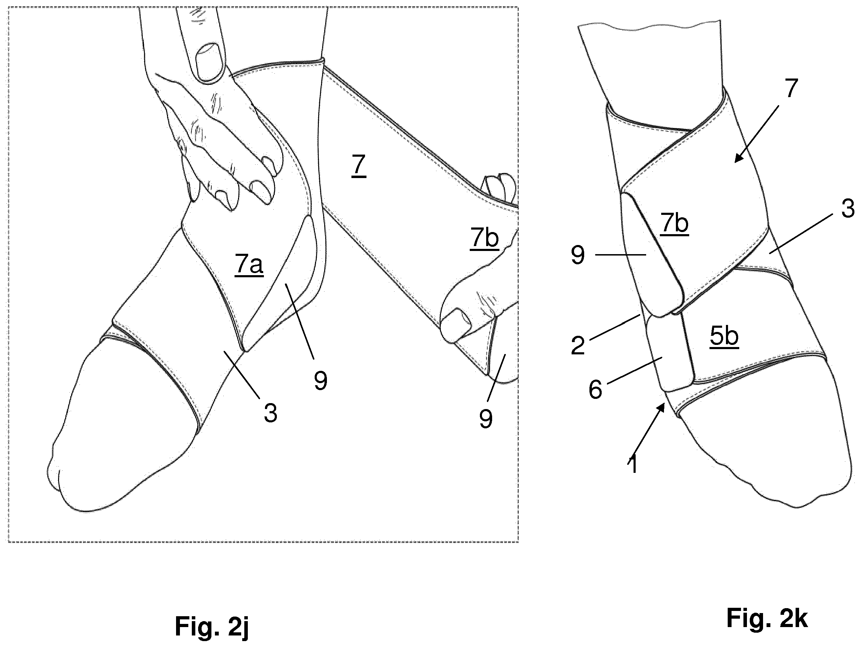

Der sich aus dem Sohlenabschnitt 1 und den Seitenabschnitten 2 und 3 mit den distalen Laschenabschnitten 4, 5 zusammensetzende Teil der Fußbandage kann mittels der Befestigungsmittel 6 an dem Fuß eines Patienten so unter Zug befestigt werden, dass dieser Teil der Fußbandage einen Kompressionsdruck auf den Fuß ausübt. Der bei angelegter Fußbandage auf den Fuß ausgeübte Kompressionsdruck liegt - je nach eingestellter Dehnung - zweckmäßig im Bereich zwischen 8 und über 40 mmHg und kann auf Kompressionswerte bis zu 60 mmHg eingestellt werden. Die Dehnung des elastischen Materials erfolgt dabei durch manuellen Zug an den distalen Laschenabschnitten 4, 5 und wird durch die Befestigung der Befestigungsmittel 6 fixiert. Hierfür werden die distalen Laschenabschnitte 4, 5 mit den daran angeordneten Zuglaschen 4a, 5b bzw. 5a, 5b um den Fußrücken gewickelt und der distale Laschenabschnitt 5, an dessen Zuglaschen 5a, 5b die Befestigungsmittel 6 angeordnet sind, wird unter Zug mittels der Befestigungsmittel 6 auf dem gegenüberliegenden Seitenabschnitt 2 befestigt. Das Anlegen der Fußbandage an einen Fuß wird nachfolgend unter Bezugnahme auf Figur 2 noch im Einzelnen erläutert.

Die erfindungsgemäße Fußbandage umfasst weiterhin ein abnehmbares proximales Kompressionsband 7. Dieses Kompressionsband 7 ist in der Darstellung der Figur 1 in geöffnetem Zustand und in der Darstellung der Figur 1b in geschlossenem Zustand gezeigt. Das proximale Kompressionsband 7 ist bei angelegter Fußbandage abnehmbar an den Seitenabschnitten 2, 3 befestigt. Wie der Sohlenabschnitt 1 und die Seitenabschnitte 2 und 3 mit den distalen Laschenabschnitten 4, 5 ist auch das proximale Kompressionsband 7 aus einem elastischen Material gebildet, zweckmäßig aus demselben elastischen Material wie der Sohlenabschnitt und die beiden Seitenabschnitte mit den Laschenabschnitten. Zur abnehmbaren Befestigung des proximalen Kompressionsbands 7 an den Seitenabschnitten 2, 3 verfügt das proximale Kompressionsband 7 über ein erstes Befestigungsmittel 8. Die ersten Befestigungsmittel 8 sind zweckmäßig durch ein Hakenteil eines Klettverschlusses gebildet, welches an einer Innenseite des Kompressionsbands 7 an einer zentralen Stelle befestigt, insbesondere aufgenäht ist. Das erste Befestigungsmittel 8 ist in der Darstellung der Figur 1a erkennbar. Mittels des ersten Befestigungsmittels 8 wird das proximale Kompressionsband 7 in dem Bereich 2a, 3a oberhalb der Ferse auf der Außenseite der Seitenabschnitte 2, 3 befestigt. Zur Ausübung eines Kompressionsdrucks wird das proximale Kompressionsband 7 um einen Unterschenkelabschnitt des Patienten geschlungen und dort mittels eines zweiten Befestigungsmittels 9 unter Dehnung des proximalen Kompressionsbands 7 befestigt. Das proximale Kompressionsband 7 weist dabei einen ersten Endabschnitt 7a und einen zweiten Endabschnitt 7b auf, wobei wenigstens an einem der beiden Endabschnitte 7a das zweite Befestigungsmittel 9 vorgesehen sind. Bei dem zweiten Befestigungsmittel 9 handelt es sich zweckmäßig wiederum um einen Klettverschluss und insbesondere um ein abnehmbar an dem Endabschnitt 7a befestigtes Hakenteil eines Klettverschlusses.

Nachfolgend wird unter Bezugnahme auf Figur 2 das Anlegen der Fußbandage an einen Fuß eines Patienten erläutert. Hierzu wird zunächst der aus dem Sohlenabschnitt 1 und den beiden Seitenabschnitten 2 und 3 mit den distalen Laschenabschnitten 4, 5 bestehende Teil der Fußbandage am Fuß des Patienten angelegt, wie in Figur 2a dargestellt, wobei der Sohlenabschnitt 1 im Bereich der Fußsohle zu liegen kommt und die beiden Seitenabschnitte 2, 3 mit den daran angeordneten distalen Laschenabschnitten 4, 5 um den Fußrücken gewickelt werden. Die Vertikalnaht 10, durch welche die beiden Seitenabschnitte 2, 3 miteinander verbunden sind, kommt dabei im Bereich der Achillessehne zu liegen und die Vertikalnaht 10 verläuft im Wesentlichen in vertikaler Richtung. Die Ausnehmung 13 liegt im Bereich der Ferse des Patienten.

Durch manuellen Zug an den Zuglaschen 4a, 4b bzw. 5a, 5b der distalen Laschenabschnitte 4, 5 wird eine Kompression auf den Fuß des Patienten ausgeübt. Diese Kompression wird durch Festlegen der gezogenen Laschenabschnitte 4, 5 am jeweils gegenüberliegenden Seitenabschnitt 2, 3 fixiert. Hierfür werden die an den freien Enden der Zuglaschen 5a, 5b des einen Laschenabschnitts 5 angeordnete Befestigungsmittel 6 auf der Außenseite des gegenüberliegenden Seitenabschnitts 2 fixiert. Die Befestigungsmittel 6 sind dabei zweckmäßig abnehmbar ausgebildet. Dies ermöglicht es, die Fußbandage sowohl für einen rechten als auch einen linken Fuß einzusetzen. Das Befestigen der Befestigungsmittel 6 an den freien Enden der Zuglaschen 5a, 5b des eines distalen Laschenabschnitts 5 ist in Figur 2b gezeigt. Bei den Befestigungsmitteln 6 handelt es sich um Klettverschlüsse, deren Hakenteil mit einem Abschnitt auf der Außenseite des freien Endes der Zuglaschen 5a, 5b angeklettet werden. Ein anderer Abschnitt der Befestigungsmittel 6 bleibt dabei frei, damit dieser auf der Außenseite des gegenüberliegenden Seitenabschnitts 2 angeklettet werden kann.

Zur Fixierung der Fußbandage am Fuß wird zunächst eine erste Zuglasche 5b und danach die zweite Zuglasche 5a des einen distalen Laschenabschnitts 5 mittels der Befestigungsmittel 6 an der Außenseite des gegenüberliegenden Seitenabschnitts 2 befestigt, wie in den Figuren 2c, 2d und 2e gezeigt. Dabei wird über die Zuglaschen 5a, 5b ein Zug auf den Laschenabschnitt 5 ausgeübt, der sich auf den Seitenabschnitt 3 und den Sohlenabschnitt 1 überträgt. Durch die Festlegung der Befestigungsmittel 6 der Zuglaschen 5a, 5b an dem gegenüberliegenden Seitenabschnitt 2 wird die durch den Zug auf das elastische Material ausgeübte Dehnung fixiert, so dass eine Kompression auf den Fuß des Patienten ausgeübt wird.

Danach wird das proximale Kompressionsband 7 im Bereich des Unterschenkels des Patienten befestigt. Hierfür wird zunächst die Innenseite des proximalen Kompressionsbands 7 mittels des ersten Befestigungsmittels 8 auf der Außenseite der Seitenabschnitte 2, 3 befestigt. Die Befestigung erfolgt dabei in dem Bereich 2a, 3a der Seitenabschnitte 2, 3, der oberhalb der Ferse liegt. Die Befestigung des proximalen Kompressionsbands 7 an den Seitenabschnitten 2, 3 über das erste Befestigungsmittel 8 ist in Figur 2f dargestellt. Die genaue Lage des proximalen Kompressionsbands 7 kann dabei den individuellen Erfordernissen der Kompressionstherapie entsprechend angepasst werden, wobei das proximale Kompressionsband 7 insbesondere in proximal-distaler Richtung verschoben werden kann. Das proximale Kompressionsband 7 kann daher insbesondere in einem Unterschenkelabschnitt oder auch in dem Bereich um das Sprunggelenk des Patienten angelegt und befestigt werden.

Zum Festlegen des proximalen Kompressionsbands 7 an einer gewünschten Stelle am Unterschenkel bzw. am Sprunggelenk des Patienten werden zunächst, wie in Figur 2g gezeigt, an einem der Endabschnitte 7a des proximalen Kompressionsbands 7 abnehmbare zweite Befestigungsmittel 9 angeordnet. Bei den zweiten Befestigungsmitteln 9 handelt es sich zweckmäßig um Hakenteile eines Klettverschlusses, welche auf der Außenseite des proximalen Kompressionsbands 7 im Bereich eines der Endabschnitte 7a festgeklettet werden. Zweckmäßig wird, wie in Figur 2h gezeigt, auch an dem anderen Endabschnitt 7b des proximalen Kompressionsbands 7 ein zweites Befestigungsmittel 9 angeordnet. Das proximale Kompressionsband 7 wird danach, wie in Figur 2j gezeigt, mittels der zweiten Befestigungsmittel 9 zunächst an einem Endabschnitt 7a auf der Außenseite eines Seitenabschnitts 3 der Fußbandage befestigt. Danach wird auch der andere Endabschnitt 7b des proximalen Kompressionsbands 7 an dem gegenüberliegenden Seitenabschnitt 2 der Fußbandage mittels der zweiten Befestigungsmittel 9 befestigt. Dabei wird das proximale Kompressionsband 7 manuell unter Zug gehalten und dieser Zug wird durch Festlegen der zweiten Befestigungsmittel 9 fixiert, so dass das proximale Kompressionsband 7 ebenfalls eine Kompression auf den Unterschenkel oder auf den Fuß des Patienten im Bereich des Sprunggelenks ausübt. In Figur 2k ist die angelegte Fußbandage in einer Draufsicht auf den Fuß des Patienten gezeigt.

Beim Befestigen der Laschenabschnitte 4, 5 an dem jeweils gegenüberliegenden Seitenabschnitt 3 bzw. 2 ist darauf zu achten, dass der Fuß des Patienten vollständig von den Seitenabschnitt 2 und 3 und den Laschenabschnitten 4, 5 umgriffen wird, so dass keine freien Stellen entstehen, durch die Körpergewebe durchquellen könnte. In entsprechender Weise sollte auch das proximale Kompressionsband 7 so angelegt werden, dass es zumindest teilweise mit den Seitenabschnitten 2, 3 überlappt.

Die Erfindung ist nicht auf die hier zeichnerisch dargestellte Ausführungsform beschränkt. So kann beispielsweise die Anzahl der Zuglaschen der Laschenabschnitte 4, 5 an die Größe der Bandage bzw. die Anatomie des Fußes angepasst werden. Bei längeren Bandagen kann jeder Laschenabschnitt 4, 5 bspw. auch drei oder mehr Zuglaschen umfassen und bei kürzeren Bandagen kann auch nur eine Zuglasche ausreichend sein. Weiterhin ist es ausreichend, wenn nur an einem der beiden Seitenteile 2, 3 ein Laschenabschnitt mit wenigstens einer Zuglasche angeordnet ist. Die hier dargestellte Ausführungsform der Erfindung mit Laschenabschnitten 4, 5 an beiden Seitenteilen 2, 3 weist jedoch den Vorteil auf, dass die Fußbandage sowohl für einen rechten als auch für einen linken Fuß verwendet werden kann.

Die Dehnungscharakteristik der elastischen Materialen ist in weiten Bereichen variabel und kann den Erfordernissen der mit der Bandage durchzuführenden Kompressionsbehandlung angepasst werden. Weiterhin können statt Klettverschlüssen andere Befestigungsmittel zum Einsatz kommen, wie z.B. Druckknöpfe oder Haken- und Ösen-Verbindungen.

## Patentansprüche

1. Fußbandage zur Kompressionstherapie von Lymphödemen am Fuß, mit einem Sohlenabschnitt (1), sich an den Sohlenabschnitt (1) seitlich anschließenden Seitenabschnitten (2, 3), welche zumindest in einem bei angelegter Fußbandage oberhalb der Ferse zu liegen kommenden Abschnitt (2a, 3a) miteinander verbunden sind, und wenigstens einem distalen Laschenabschnitt (4, 5) aus einem elastischen Material, der um den Fußrücken wickelbar und an einem Seitenabschnitt (2, 3) befestigbar ist, um eine Kompression auf den Fuß auszuüben, wobei der oder jeder distale Laschenabschnitt (4, 5) über wenigstens eine Zuglasche (4a, 4b; 5a, 5b) verfügt, an dem Befestigungsmittel (6) angeordnet sind, **gekennzeichnet durch** ein abnehmbar an den Seitenabschnitten (2, 3) befestigbares proximales Kompressionsband (7) aus einem elastischen Material, wobei das Kompressionsband (7) über erste Befestigungsmittel (8) verfügt und bei angelegter Fußbandage in dem Bereich (2a, 3a) oberhalb der Ferse mittels der ersten Befestigungsmittel (8) an den Seitenabschnitten (2, 3) befestigt ist.

2. Fußbandage nach Anspruch 1, **dadurch gekennzeichnet, dass** das proximale Kompressionsband (7) einen ersten Endabschnitt (7a) und einen zweiten Endabschnitt (7b) sowie wenigstens an einem der Endabschnitte (7a, 7b) angeordnete zweite Befestigungsmittel (9) aufweist und bei angelegter Fußbandage um einen distalen Unterschenkelabschnitt gewickelt und dort unter Dehnung mittels der zweiten Befestigungsmittel (9) festgelegt ist.

3. Fußbandage nach Anspruch 1 oder 2, **dadurch gekennzeichnet, dass** jeder Seitenabschnitt (2, 3) über einen distalen Laschenabschnitt (4, 5) verfügt.

4. Fußbandage nach Anspruch 3, **dadurch gekennzeichnet, dass** jeder distale Laschenabschnitt (4, 5) über eine erste Zuglasche (4a, 5a) und mindestens eine zweite Zuglasche (4b, 5b) verfügt.

5. Fußbandage nach Anspruch 4, **dadurch gekennzeichnet, dass** an jeder Zuglasche (4a, 4b; 5a, 5b) des ersten Laschenabschnitts (4) ein Befestigungsmittel (6) zum lösbaren Befestigen der jeweiligen Zuglasche (4a, 4b) an dem Laschenabschnitt (5) und insbesondere einer Zuglasche (5a, 5b) des gegenüberliegenden Seitenabschnitts (3) angeordnet ist.

6. Fußbandage nach Anspruch 1 oder 4, **dadurch gekennzeichnet, dass** die Befestigungsmittel (6) lösbar an dem jeweiligen distalen Laschenabschnitt (4, 5) und insbesondere lösbar an den Zuglaschen (4a, 4b; 5a, 5b) des jeweiligen Laschenabschnitts (4, 5) angeordnet sind.

7. Fußbandage nach einem der Ansprüche 4 bis 6, **dadurch gekennzeichnet, dass** benachbarte Zuglaschen (4a, 4b; 5a 5b) eines distalen Laschenabschnitts (4; 5) durch einen Einschnitt (12) im jeweiligen Laschenabschnitt (4; 5) voneinander getrennt sind.

8. Fußbandage nach einem der voranstehenden Ansprüchen, **dadurch gekennzeichnet, dass** die Seitenabschnitte (2, 3) aus demselben elastischen Material sind wie die distalen Laschenabschnitte (4, 5).

9. Fußbandage nach einem der voranstehenden Ansprüchen, **dadurch gekennzeichnet, dass** der oder jeder distale Laschenabschnitt (4, 5) einstückig mit einem der Seitenabschnitte (2, 3) verbunden ist.

10. Fußbandage nach einem der voranstehenden Ansprüchen, **dadurch gekennzeichnet, dass** die Seitenabschnitte (2, 3) längs einer Vertikalnaht (10) miteinander verbunden sind, wobei die Vertikalnaht (10) bei angelegter Fußbandage im Bereich der Achillessehne verläuft und/oder dass jeder Seitenabschnitt (2, 3) längs einer Horizontalnaht (11) mit dem Sohlenabschnitt (1) verbunden ist.

11. Fußbandage nach einem der voranstehenden Ansprüchen, **dadurch gekennzeichnet, dass** jeder Seitenabschnitt (2, 3) über eine proximale Kante (2b, 3b) und über eine distale Kante (2c, 3c) verfügt und dass jeweils zwischen der proximalen Kante (2b, 3b) und der distalen Kante (2c, 3c) ein Laschenabschnitt (4, 5) angeordnet ist.

12. Fußbandage nach Anspruch 14, **dadurch gekennzeichnet, dass** sich die Verlängerungen der proximalen Kante (2b, 3b) und der distalen Kante (2c, 3c) jedes Seitenabschnitts (2, 3) unter einem Winkel von 40° bis 50° und insbesondere unter einem 45°-Winkel treffen.

13. Fußbandage nach einem der voranstehenden Ansprüchen, **dadurch gekennzeichnet, dass** im Fersenbereich eine Ausnehmung (13) vorgesehen ist und/oder dass im Sohlenabschnitt (1) eine Polsterung eingearbeitet, insbesondere auf einer Außenseite des Sohlenabschnitts (1) aufgenäht, ist.

14. Fußbandage nach einem der voranstehenden Ansprüchen, **dadurch gekennzeichnet, dass** es sich bei den Befestigungsmitteln (6, 8, 9) jeweils um Klettverschlüsse handelt, wobei das Hakenteil der Klettverschlüsse bevorzugt abnehmbar an den Zuglaschen (4a, 4b; 5a, 5b) der distalen Laschenabschnitte bzw. am proximalen Kompressionsband (7) befestigt sind.

15. Fußbandage nach einem der voranstehenden Ansprüchen, **dadurch gekennzeichnet, dass** das proximale Kompressionsband (7) mittels der ersten Befestigungsmittel (8) im Bereich des Abschnitts (2a, 3a) oberhalb der Ferse in Vertikalrichtung an unterschiedlichen Stellen an den Seitenabschnitten (2, 3) befestigbar ist und bei angelegter Fußbandage insbesondere im Bereich einer Vertikalnaht (10), welche die beiden Seitenabschnitte (2, 3) miteinander verbindet, lösbar auf der Außenseite der beiden Seitenabschnitte (2, 3) befestigt ist.

## Claims

1. Foot bandage for compression treatment of lymphedemas on the foot, having a sole section (1), side sections (2, 3) laterally adjoining the sole section (1) which are joined to one another at least in a section (2a, 3a) coming to rest above the heel when the foot bandage is applied, and at least one distal strap section (4, 5) made of an elastic material which can be wound around the instep and is attachable to a side section (2, 3) in order to exert a compression on the foot, wherein the or each distal strap section (4, 5) has at least one pull strap (4a, 4b; 5a, 5b), on which attachment means (6) are arranged, **characterised by** a proximal compression band (7) made of an elastic material which is attachable to the side sections (2, 3) to be removable, wherein the compression band (7) has first attachment means (8) and when the foot bandage is applied, is attached to the side sections (2, 3) by means of the first attachment means (8) in the region (2a, 3a) above the heel.

2. Foot bandage according to claim 1, **characterised in that** the proximal compression band (7) has a first end section (7a) and a second end section (7b) as well as at least second attachment means (9) arranged on one of the end sections (7a, 7b) and when the foot bandage is applied, is wound around a distal lower leg section and fixed there with stretching by means of the second attachment means (9).

3. Foot bandage according to claim 1 or 2, **characterised in that** each side section (2, 3) has a distal strap section (4, 5).

4. Foot bandage according to claim 3, **characterised in that** each distal strap section (4, 5) has a first pull strap (4a, 5a) and at least one second pull strap (4b, 5b).

5. Foot bandage according to claim 4, **characterised in that** an attachment means (6) for releasable attachment of the respective pull strap (4a, 4b) to the strap section (5) and in particular of a pull strap (5a, 5b) of the opposite side section (3) is arranged on each pull strap (4a, 4b; 5a, 5b) of the first strap section (4).

6. Foot bandage according to claim 1 or 4, **characterised in that** the attachment means (6) are arranged to be releasable on the respective distal strap section (4, 5) and in particular to be releasable on the pull straps (4a, 4b; 5a, 5b) of the respective strap section (4, 5).

7. Foot bandage according to one of claims 4 to 6, **characterised in that** adjacent pull straps (4a, 4b; 5a, 5b) of a distal strap section (4; 5) are separated from one another by an indentation (12) in the respective strap section (4; 5).

8. Foot bandage according to one of the preceding claims, **characterised in that** the side sections (2, 3) are made of the same elastic material as the distal strap sections (4, 5).

9. Foot bandage according to one of the preceding claims, **characterised in that** the or each distal strap section (4, 5) is joined in one piece to one of the side sections (2, 3).

10. Foot bandage according to one of the preceding claims, **characterised in that** the side sections (2, 3) are joined to one another along a vertical seam (10), wherein the vertical seam (10) runs in the region of the Achilles tendon when the foot bandage is applied and/or **in that** each side section (2, 3) is joined to the sole section (1) along a horizontal seam (11).

11. Foot bandage according to one of the preceding claims, **characterised in that** each side section (2, 3) has a proximal edge (2b, 3b) and a distal edge (2c, 3c) and **in that** a strap section (4, 5) is arranged respectively between the proximal edge (2b, 3b) and the distal edge (2c, 3c).

12. Foot bandage according to claim 14, **characterised in that** the extensions of the proximal edge (2b, 3b) and of the distal edge (2c, 3c) of each side section (2, 3) meet at an angle of 40° to 50° and in particular at a 45° angle.

13. Foot bandage according to one of the preceding claims, **characterised in that** a recess (13) is provided in the heel region and/or **in that** padding is incorporated in the sole section (1), in particular sewn on an outer side of the sole section (1).

14. Foot bandage according to one of the preceding claims, **characterised in that** the attachment means (6, 8, 9) are respectively Velcro closures, wherein the hook part of the Velcro closures are attached to the pull straps (4a, 4b; 5a, 5b) of the distal strap sections or to the proximal compression band (7) preferably to be removable.

15. Foot bandage according to one of the preceding claims, **characterised in that** the proximal compression band (7) is attachable by means of the first attachment means (8) in the region of the section (2a, 3a) above the heel in vertical direction at different points on the side sections (2, 3) and when the foot bandage is applied, is attached in particular in the region of a vertical seam (10), which joins the two side sections (2, 3) to one another, to be releasable on the outer side of the two side sections (2, 3).

## Revendications

1. Bandage pour pied destiné à la thérapie par compression de lymphœdèmes au niveau du pied, avec une section de semelle (1), des sections latérales (2, 3) se raccordant latéralement à la section de semelle (1), lesquelles sont reliées les unes aux autres au moins dans une section (2a, 3a) venant se placer à l'horizontale au-dessus du talon lorsque le bandage pour pied est placé, et au moins une section de bride distale (4, 5) composée d'un matériau élastique, qui peut être enroulée autour de la partie arrière du pied et peut être fixée au niveau d'une section latérale (2, 3) pour exercer une compression sur le pied, dans lequel la ou chaque section de bride distale (4, 5) dispose d'au moins une bride de traction (4a, 4b ; 5a, 5b), au niveau de laquelle sont disposés des moyens de fixation (6), **caractérisé par** une bande de compression proximale (7) pouvant être fixée de manière à pouvoir être retirée au niveau des sections latérales (2, 3), composée d'un matériau élastique, dans lequel la bande de compression (7) dispose de premiers moyens de fixation (8) et est fixée au niveau des sections latérales (2, 3) au moyen des premiers moyens de fixation (8) dans la zone (2a, 3a) au-dessus du talon lorsque le bandage pour pied est placé.

2. Bandage pour pied selon la revendication 1, **caractérisé en ce que** la bande de compression proximale (7) présente une première section d'extrémité (7a) et une deuxième section d'extrémité (7b) ainsi qu'au moins des deuxièmes moyens de fixation (9) disposés au niveau d'une des sections d'extrémité (7a, 7b) et est enroulée autour d'une section de jambe distale lorsque le bandage pour pied est placé et y est fixée moyennant un allongement au moyen des deuxièmes moyens de fixation (9) .

3. Bandage pour pied selon la revendication 1 ou 2, **caractérisé en ce que** chaque section latérale (2, 3) dispose d'une section de bride distale (4, 5).

4. Bandage pour pied selon la revendication 3, **caractérisé en ce que** chaque section de bride distale (4, 5) dispose d'une première bride de traction (4a, 5a) et d'au moins une deuxième bride de traction (4b, 5b).

5. Bandage pour pied selon la revendication 4, **caractérisé en ce qu'**un moyen de fixation (6) pour fixer de manière amovible la bride de traction (4a, 5b) respective au niveau de la section de bride (5) et en particulier une bride de traction (5a, 5b) de la section latérale faisant face (3) est disposé au niveau de chaque bride de traction (4a, 4b ; 5a, 5b) de la première section de bride (4).

6. Bandage pour pied selon la revendication 1 ou 4, **caractérisé en ce que** les moyens de fixation (6) sont disposés de manière amovible au niveau de la section de bride distale (4, 5) respective et en particulier de manière amovible au niveau des brides de traction (4a, 4b ; 5a, 5b) de la section de bride (4, 5) respective.

7. Bandage pour pied selon l'une quelconque des revendications 4 à 6, **caractérisé en ce que** des brides de traction (4a, 4b ; 5a, 5b) adjacentes d'une section de bride distale (4 ; 5) sont séparées les unes des autres par une incision (12) dans la section de bride (4 ; 5) respective.

8. Bandage pour pied selon l'une quelconque des revendications précédentes, **caractérisé en ce que** les sections latérales (2, 3) sont composées du même matériau élastique que les sections de bride distales (4, 5).

9. Bandage pour pied selon l'une quelconque des revendications précédentes, **caractérisé en ce que** la ou chaque section de bride distale (4, 5) est reliée d'un seul tenant à une des sections latérales (2, 3).

10. Bandage pour pied selon l'une quelconque des revendications précédentes, **caractérisé en ce que** les sections latérales (2, 3) sont reliées les unes aux autres le long d'une couture verticale (10), dans lequel la couture verticale (10) s'étend dans la zone du tendon d'Achille lorsque le bandage pour pied est placé, et/ou que chaque section latérale (2, 3) est reliée à la section de semelle (1) le long d'une couture horizontale (11).

11. Bandage pour pied selon l'une quelconque des revendications précédentes, **caractérisé en ce que** chaque section latérale (2, 3) dispose d'une arête proximale (2b, 3b) et d'une arête distale (2c, 3c), et qu'une section de bride (4, 5) est disposée respectivement entre l'arête proximale (2b, 3b) et l'arête distale (2c, 3c).

12. Bandage pour pied selon la revendication 14, **caractérisé en ce que** les prolongements de l'arête proximale (2b, 3b) et de l'arête distale (2c, 3c) de chaque section latérale (2, 3) se rencontrent selon un angle de 40° à 50° et en particulier selon un angle de 45°.

13. Bandage pour pied selon l'une quelconque des revendications précédentes, **caractérisé en ce qu'**un évidement (13) est prévu dans la zone de talon, et/ou qu'un rembourrage est pratiqué, en particulier est cousu sur un côté extérieur de la section de semelle (1) dans la section de semelle (1).

14. Bandage pour pied selon l'une quelconque des revendications précédentes, **caractérisé en ce que** les moyens de fixation (6, 8, 9) sont respectivement des fermetures auto-agrippantes, dans lequel la partie de crochet des fermetures auto-agrippantes est fixée de manière préférée de manière à pouvoir être retirée au niveau des brides de traction (4a, 4b ; 5a, 5b) des sections de bride distales ou au niveau de la bande de compression proximale (7).

15. Bandage pour pied selon l'une quelconque des revendications précédentes, **caractérisé en ce que** la bande de compression proximale (7) peut être fixée au niveau des sections latérales (2, 3) au niveau d'emplacements différents dans la direction verticale au-dessus du talon dans la zone de la section (2a, 3a) au moyen des premiers moyens de fixation (8) et est fixée de manière amovible sur le côté extérieur des deux sections latérales (2, 3) en particulier dans la zone d'une couture verticale (10), laquelle relie entre elles les deux sections latérales (2, 3) lorsque le bandage pour pied est placé.
